# EUROPÄISCHE PATENTSCHRIFT

(11) **EP 4 268 119 B1**
(45) Veröffentlichungstag und Bekanntmachung des Hinweises auf die Patenterteilung: **01.05.2024**
(21) Anmeldenummer: 22761972.3
(22) Anmeldetag: 02.08.2022
(51) Int. Cl.: G06K 7/00, G06K 7/016, A61B 90/90, A61B 90/96, A61B 90/98, A61B 90/92

(54) **SMART PORT - MULTIFUNKTIONALES LESE/ERKENNUNGSGERÄT IM STERILGUTKREISLAUF**
SMART-PORT MULTIFUNCTIONAL READER/IDENTIFIER IN A PRODUCT-STERILISATION CYCLE
PORT INTELLIGENT - DISPOSITIF DE LECTURE/RECONNAISSANCE MULTIFONCTIONNEL DANS UN CIRCUIT DE PRODUIT STÉRILE

(30) Priorität: 04.08.2021 DE 102021120340
(43) Veröffentlichungstag der Anmeldung: 01.11.2023
(73) Patentinhaber: Aesculap AG, 78532 Tuttlingen (DE)
(72) Erfinder: LENZENHUBER, Frederick, 78532 Tuttlingen (DE); HÖGERLE, Roland-Alois, 78532 Tuttlingen (DE)
(74) Vertreter: Winter, Brandl - Partnerschaft mbB
(86) Internationale Anmeldenummer: PCT/EP2022/071746
(87) Internationale Veröffentlichungsnummer: WO 2023/012184

(56) Entgegenhaltungen:
- EP-A2- 2 234 032
- WO-A1-2020/200975
- KR-A- 20140 100 776
- US-A1- 2007 210 159
- US-A1- 2011 156 869
- US-A1- 2018 214 243
- US-B2- 9 501 671
- US-B2- 10 117 722

## Beschreibung

### Technisches Gebiet

Die vorliegende Offenbarung betrifft einen Smart Port zum Erfassen und Auslesen von medizinischen Produkten mittels einer Vielzahl an, vorzugsweise modularen Modulen, welche jeweils mit unterschiedlichen Erfassungstechnologien vorgesehen und ausgebildet sind. Ferner betrifft die vorliegende Offenbarung ein System mit einem Smart Port, einem Siebkorb und darin einbringbaren Instrumentenhaltern, welche die medizinischen Produkte halten. Ferner betrifft die vorliegende Offenbarung einen Roboterarm mit einem daran angebrachten oder darin integrierten Smart Port sowie eine Vorrichtung mit einer künstlichen Intelligenz.

### Hintergrund der Erfindung

Mit einem Lese- und/ oder Schreibgerät können aktuell am Markt befindliche Produktkennzeichnungen / Produktdatenträger von verschiedenen medizinischen Produkten gelesen und ggf. beschrieben werden. Stand heute haben die Kliniken verschiedenste Lösungen, um die medizinischen Produkte zu markieren.

Diese Produktmarkierungen haben den Hintergrund, dass in einer Klinik beispielsweise der Sterilisationszyklus bestmöglich nachvollziehbar und aber auch nachweisbar sein soll. Durch eine lückenlose Dokumentation über den Verbleib, den Verschleiß, das Alter, den Zustand, Wartungen etc. ist es den Kliniken möglich, beispielsweise im Fall einer Patientenschädigung, nachzuweisen, dass entsprechend verwendete medizinische Produkte in einem einwandfreien Zustand waren.

Die Produktbeschriftungen wie Artikelnummer und Seriennummer sind aktuell direkt (händisch) mittels einem Data Matrix Code (GTIN), auch als QR-Code bekannt, einem Barcode und vereinzelt mittels RFID Tags zu erfassen. Letztere kommen Stand heute noch sehr selten zum Einsatz, da die Vielfältigkeit der Tags! Chips die Kliniken vor Herausforderungen stellt. Zu jeder Technologie der Produktbeschriftung ist ein gesondertes Lese- und/ oder Schreibgerät erforderlich, welches wiederum Informationen über die entsprechenden medizinischen Produkte an eine Vielzahl von Instrumentendatenmanagementsysteme weiterreicht/ weiterleitet.

Unter medizinischen Produkten werden beispielsweise chirurgische Instrumente sowie sämtliche Produkte, welche zu sterilisieren sind, verstanden.

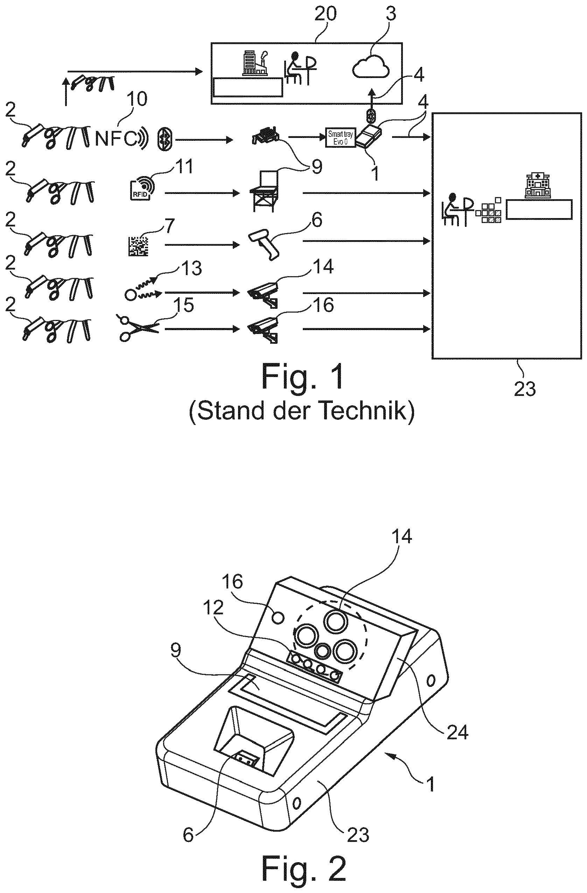

Fig. 1 zeigt gemäß dem aktuellen Stand der Technik medizinische Produkte 2 aus eigener Herstellung, welche mit NFC-Technologie/ einem NFC-Tag 10 ausgestattet sind und über eine intelligente Steuereinheit mit und ohne Multiplexer ausgelesen werden können. Zudem können die NFC-Tags über einen Smart Port 1 bzw. ein Lese- und/ oder Schreibegerät ausgelesen werden, welcher stand heute schone eine NFC-Schnittstelle 4 zur Verfügung stellt.

Fremde medizinische Produkte 2 haben hingegen andere Technologien verbaut. Hierunter zählen beispielsweise die in Fig. 1 unter der NFC-Technologie abgebildeten Möglichkeiten, wie RFID-NFC, RDIF-LF, RFID-HF, RFID-UHF, RFID-SHF 11, und weitere, als bidirektionaler Speicher, aber auch ein Data Matrix Code 7, ein Barcode 8, Quantumdots, und weitere, als fester Datenspeicher. Gegebenenfalls sind auch Kabelanschlüsse denkbar, wobei diese hier nicht weiter beschrieben werden.

In anderen Worten bedeutet das, dass fremde medizinische Produkte über gesonderte Lesegeräte oder geeignete Erfassungssystem geloggt werden. Dies erhöht das Vorhallten von Hardware und mach die Handhabung sehr kompliziert, so dass Schulungen und Einweisungen auf jedwede Hardware stattfinden muss.

### Stand der Technik

In EP 0 630 820 B1 ist ein Verfahren zum Überwachen des Materialflusses bei der Aufbereitung von Sterilgutbehandlung offenbart, mit folgenden Schritten: Sterilgut wird in einen Sterilgutbehälter verpackt. Die so gebildete Sterilisationseinheit charakterisierende Daten werden aufgezeichnet und auf einem mit der Sterilisationseinheit verbindbaren Datenträger gespeichert. Die Sterilisationseinheit wird in einem Sterilisator sterilisiert und die den Sterilisationsvorgang charakterisierenden Daten werden erfasst und registriert. Die Sterilisationseinheit charakterisierende Daten werden in eine an den Sterilisator angeschlossene Datenverarbeitungseinrichtung eingegeben. In Abhängigkeit von die Sterilisationseinheit charakterisierenden Daten wird ein geeignetes Sterilisationsverfahren ausgewählt. Die die Sterilisationseinheit und den Sterilisationsvorgang charakterisierenden Daten werden gemeinsam gespeichert.

US 10 783 991 A1 beschreibt ein System, das eine Vielzahl von RFID-Chips, die an einem Katheter befestigt sind, eine Datenerfassungsmaschinenvorrichtung und eine Servervorrichtung umfasst. Die Datenerfassungsmaschine überträgt drahtlos Strom an einen ersten der RFID-Chips und empfängt erste medizinische Daten von dem ersten RFID-Chip, während der erste RFID-Chip durch den Stromempfänger aktiviert wird. Die Datenerfassungsvorrichtung erzeugt eine erste Nachricht, die die ersten medizinischen Daten anzeigt, die an das Servervorrichtung zu senden sind. Die Servervorrichtung kann Aspekte des Katheters wie Position und Risikozustände basierend auf den ersten medizinischen Daten bestimmen.

In EP 1 402 904 B1 ist ein System zur Aufbereitung von medizinischen Instrumenten in einer zentralen Sterilisationsabteilung beschrieben, wobei die Instrumente mit einem Identifikationscode versehen sind, der in einem Verpackungsbereich der Sterilisationsabteilung zum Verpacken und Screenen verwendet wird und der zur Aktualisierung einer zentralen Datenbank verwendet wird.

WO 2020/200975 A1 offenbart ein System und eine Vorrichtung zum Lesen eines an einem biologischen oder pharmazeutischen Produkt angebrachten optischen Etiketts über ein Gerät, das ein optisches Lesegerät mit Computer-Vision-Funktionen und ein RFID-Tag über ein RFID-Lesegerät aufweist.

US 9 501 671 B2 offenbart ein Lesegerät mit einen Körper und einem ScanElement, wobei das Scanelement ein Barcode-Lesemodul und ein RFID-Lesemodul aufweist.

KR 2014 0100776 A offenbart ein komplexes Lesegerät mit ein 1-D-Barcode-Lesegerät, ein 2D-Barcode-Lesegerät, das einen 2D-Barcode und ein RFID-Tag-Lesegerät.

EP 2 234 032 A2 offenbart ein Gerät mit eine Auslesebox, wobei in einer Seitenfläche der Auslesebox ein Barcodescanner integriert ist und in einer anderen Seitenfläche der Auslesebox ein Radiofrequenz-Identifikationsgerät (RFID) oder ein Nahfeldkommunikationsleser (NFC) integriert ist. Weiterer relevanter Stand der Technik ist zu finden in US 10117722 B2, US 2018214243 A1, US 2011156869 A1 und US 2007210159 A1.

### Kurzbeschreibung der Offenbarung

Demnach besteht die Aufgabe der vorliegenden Offenbarung darin, dass durch die Verwendung eines Smart Ports bzw. eines multifunktionalen Lese- und/ oder Schreibgeräts zum einen Fehler in der Dokumentation vermieden, als auch der Zeitaufwand/ Arbeitsaufwand reduziert werden. Ferner ist es Ziel der vorliegenden Offenbarung, dass weniger Hardware benötigt wird, um die eigenen als auch die fremden medizinischen Produkte zu erfassen.

Die vorstehende Aufgabe wird gelöst durch ein System mit einem Smart Port /einem multifunktionales Lese- und/ oder Schreibegerät mit den Merkmalen des Patentanspruchs 1.

Der Kern der Offenbarung besteht demzufolge darin, dass der Smart Port/ das multifunktionale Lese- und/ oder Schreibegerät zum Erfassen und Auslesen von medizinischen Produkten mittels einer Vielzahl an, vorzugsweise modularen Modulen, bereitgestellt wird, wobei die Vielzahl an Module jeweils mit unterschiedlichen Erfassungstechnologien (ggf. mit zueinander unterschiedlichen Erfassungs-Reichweiten) vorgesehen und ausgebildet sind.

In anderen Worten bedeutet das, dass der Smart Port ein multifunktionales Gerät ist, in welchem mehrere voneinander unterschiedliche Erfassungstechnologien integrierbar bzw. integriert sind. Ein solcher Smart Port hat den Vorteil, dass zum Auslesen und/ oder Erfassen keine zusätzlichen Lesegeräte für fremde/andere medizinische Produkte notwendig sind. Der Smart Port umfasst/beinhaltet sämtliche notwendigen Erfassungstechnologien, welche den verschiedenen Produktmarkierungen entsprechend angepasst sind.

Demnach besteht die Kernerfindung darin, mehrere (zueinander unterschiedliche) Technologien miteinander in einem Gerät/ einer Vorrichtung zu kombinieren, um vorzugsweise unter Einsatz von künstlicher Intelligenz weitereichende Anwendungsfällte zu bedienen.

Hierbei werden Module und zukünftige Technologien mitbeschrieben, welche in naher Zukunft ebenso etabliert sein werden bzw. etabliert werden könnten. Auf diese Weise ist es das Ziel der vorliegenden Offenbarung, mit dem Smart Port nahezu alle Technologien abzudecken und alle Identifikationssystem bedienen zu können.

Die vorliegende Offenbarung hat den Vorteil, dass dem Personal in der CSSD ein einfach handhabbares Lese- und Schreibgerät zur Verfügung gestellt wird. Hierbei ist das Handling wesentlich einfacher und aufgrund einer Kompakthardware platzsparender. Prozesse können durch den Einsatz des Smart Ports schlanker und optimierten gestaltet werden. Zudem wird weniger Hardware benötigt und die Kernfrage bezüglich der Lebensdauer unter Zuhilfenahme von künstlicher Intelligenz kann beantwortet werden. Ferner sind Kosteneinsparungen durch eine Halb- und/oder Vollautomatisierung möglich.

Nachfolgend werden weitere bevorzugte Aspekte gemäß den Unteransprüchen näher beschrieben.

Es ist bevorzugt, wenn der Smart-Port einen um den Smart Port liegenden Raum definiert, in welchem die medizinischen Produkte auslesbar sind. In anderen Worten bedeutet das, dass um den Smart-Port ein visueller, dreidimensionaler Raum aufgespannt wird, welcher dazu vorgesehen und ausgebildet ist, um die medizinischen Produkte innerhalb dieses Raumes zu erfassen und auszulesen.

Es ist vorteilhaft, wenn der Smart Port, insbesondere eine Recheneinheit des Smart Ports oder in dem Smart Port dazu vorgesehen und ausgebildet ist, um innerhalb eines vorgegebenen und/oder vordefinierten Zeitfensters, insbesondere innerhalb von 2 bis 3 Sekunden, die medizinischen Produkte zu erfassen und ein Signal zu erzeugen. Das Signal ist dazu vorgesehen, die Protokollierung der erfassten medizinischen Geräte zu erleichtern und nahezu zeitgleich mit der Erfassung des medizinischen Geräts die richtige oder falsche Zuordnung zu protokollieren. Hierbei ist es von Vorteil, wenn innerhalb des Zeitfensters die optische Erfassungstechnologie und die Funkerfassungstechnologie gegeneinander abgleichbar sind, um die Richtigkeit der Zuordnung gewährleisten zu können.

Es ist bevorzugt, wenn der Smart Port dazu vorgesehen und ausgebildet ist, um zumindest zwei unterschiedliche Feedback-Signale, insbesondere optisch und/oder akustisch, auszugeben, wodurch eine richtige oder falsche Zuordnung erkennbar ist. Das Feedback-Signal für eine richtige Zuordnung kann beispielsweise ein kurzer Piepton und/oder grünfarbenes Aufblinken des Smart-Ports sein. Eine falsche Zuordnung kann mit einem längeren oder einem doppelten Piepton und/oder einem roten Aufblinken des Smart-Ports deutlich gemacht werden.

Gemäß einer bevorzugten Ausführungsform steht der Smart Port in vorzugsweise der Zentralen Sterilgutversorgungsabteilung (ZSVA) und/ oder der Aufbereitungseinheit für Medizinprodukte (AEMP) und/ oder der zentralen Sterilgutversorgungsabteilung (CSSD) an einem Packplatz bzw. einem zentralen Ort, an welchem jedwedes genutzte medizinische Produkt ohnehin in einem Zyklus prozessiert wird. In anderen Worten bedeutet das, dass an diesem Ort/ an dieser Stelle die medizinischen Produkte gesichtet, gepflegt und einem Siebkorb zugeordnet werden. Diese Stelle passieren alle medizinischen Produkte, welche aufbereitet und sterilisiert werden müssen.

Gemäß einer bevorzugten Ausführungsform ist der Smart Port dazu vorgesehen und ausgebildet, erfasste und ausgelesene Daten (selbst) in eine Cloud basierte Infrastruktur zu laden sowie mittels einer Schnittstelle mit einem

Instrumentendatenmanagementsystem des Kunden und/ oder des Herstellers zu kommunizieren. Für die Übertragung wird vorzugsweise WLAN, UMTS, LORAWAN (Long Range Wide Area Network) oder ähnliches verwendet.

Gemäß einer weiteren bevorzugten Ausführungsform können die einzelnen Module, welche in dem Smart Port zum Einsatz kommen, in modularer Form genutzt und weggelassen werden. Gleichzeitig kann der Smart Port auch mit neuen/ weiteren Technologien bestückt/ erweitert werden.

Gemäß einer weiteren bevorzugten Ausführungsform ist ein erstes Modul, vorzugsweise ein Scanner, dazu vorgesehen und ausgebildet, einen Data Matrix Code und/ oder einen Barcode auszulesen, welcher dazu vorgesehen und ausgebildet ist, auf einem medizinischen Produkt aufgebracht und auslesbar zu sein.

Der Data Matrix Code ist ein maschinenlesbarer Code, welcher heute Standard ist. Dieser wird auf medizinische Produkte aufgebracht und enthält GTIN (Global Trade Item Number) Informationen. Das Gerät kann alle gängigen Data Matrix Codierungen bedienen.

Der Barcode ist üblicherweise auf ein Label gedruckt und kann gescannt sowie weiterverarbeitet werden. Auch die Barcodes sind standardisiert.

Das als Scanner ausgebildete erste Modul ist eindirektional/ unidirektional ausgebildet. Demnach kann der Dateninhalt des Data Matrix Codes und des Barcodes nur ausgelesen werden. Eine Weiterverarbeitung durch ein Instrumenten-Managementsystem (IMS) oder ein weiterführendes Gerät ist möglich und vorgesehen.

Gemäß einer weiteren bevorzugten Ausführungsform ist ein zweites Modul, vorzugsweise ein RFID-Modul, insbesondere ein NFC-Modul, dazu vorgesehen und ausgebildet, zumindest einen RFID-Tag, insbesondere zumindest einen NFC-Tag auszulesen, welcher dazu vorgesehen und ausgebildet ist, in einem medizinischen Produkt untergebracht und auslesbar und/ oder beschreibbar zu sein.

Das NFC-Modul ist bevorzugt dazu vorgesehen und ausgebildet, zumindest einen NFC-Tag, welche zukünftig in Produkten zum Einsatz kommen, auszulesen, zu verarbeiten und auch wieder zu beschreiben. Hierzu ist es von Vorteil, wenn die medizinischen Produkte sehr nahe an eine Antenne angelegt werden.

Das RFID-Modul ist bevorzugt dazu vorgesehen und ausgebildet, zumindest einen RFID-Tag, welche zukünftig in medizinischen Produkten zum Einsatz kommen, auszulesen, zu verarbeiten und auch wieder zu beschreiben. Hierbei ist es von Vorteil, dass sich medizinische Produkte in einem weiteren Umfeld befinden können, als dies bei dem NFC-Modul möglich ist. Die Entfernung muss so ausgelegt sein, dass eine Software die RSSI-Werte des zumindest einen RFID-Tags erkennen und aussagen kann, wann sich ein RFID-Tag in unmittelbarer Nähe befindet. Dies ist eine Varianz.

Das als NFC-Modul und/ oder RFID-Modul ausgebildete zweite Modul ist vorzugsweise bidirektional ausgebildet. Demnach kann der Dateninhalt des NFC-Tags bzw. des RFID-Tags ausgelesen und wieder beschrieben werden. Eine Weiterverarbeitung durch ein Integriertes Managementsystem (IMS) oder ein weiterführendes Gerät ist möglich und vorgesehen.

Es ist von Vorteil, wenn das zweite Modul zudem bzw. alternativ in einem Frequenzbereich von HF, UHF und/ oder SHF arbeitet. Hierbei ist unter HF (High Frequency) eine Frequenz von etwa 3 bis 30 MHz, unter UHF (Ultra High Frequency) von etwa 0,3 bis 3 GHz und unter SHF (Super High Frequency) von etwa 3 bis 30 GHz verstanden.

Gemäß einer weiteren bevorzugten Ausführungsform ist ein drittes Modul, vorzugsweise ein LIDAR-Modul, dazu vorgesehen und ausgebildet, eine Oberflächenabtastung von zumindest einem medizinischen Produkt durchzuführen. In anderen Worten können mittels LIDAR Oberflächen, vergleichbar wie bei Radar bzw. Sonar, abgetastet werden. Dies hat den Vorteil, dass dies auch bei sehr kleinen Baugrößen funktioniert. LIDAR basiert auf Lichtimpulsen und wird unter anderem beim autonomen Fahren erfolgreich eingesetzt. Diese Technologie ist dazu vorgesehen, medizinische Produkte anhand derer Geometrie zu erkennen. Gleichzeitig können medizinische Produkte mittels dieser Technik gegenüber Sollzuständen abgeglichen werden und etwaige Defekte/ Deformationen erkannt werden. Ferner sind zum Beispiel Schneiden bei Werkzeugen scanbar und somit beurteilbar, ob diese noch intakt sind oder nicht mehr genutzt werden können.

Gemäß einer weiteren bevorzugten Ausführungsform ist ein viertes Modul, vorzugsweise ein Bilderfassungsmodul, dazu vorgesehen und ausgebildet ist, um anhand zumindest eines Bildes ein medizinisches Produkt zu erfassen. In anderen Worten können mittels dem vierten Modul medizinische Produkte anhand Ihrer Geometrie erkannt werden. Gleichzeitig können medizinische Produkte mittels dieser Technik gegenüber Sollzuständen abgeglichen werden und etwaige Defekte/Deformationen erkannt werden. Ferner sind zum Beispiel Schneiden bei Werkzeugen scanbar und somit beurteilbar, ob diese noch intakt sind oder nicht mehr genutzt werden können.

Ferner können durch Lidar und Kamera auch Produktgattungen erkannt werden. Über den Scan mittels Kamera kann auch ein DATA MATRIX Code ausgelesen werden und so Serialnummer erfasst werden.

Auf Produkten sind meist Artikelnummer und Serialnummer aufgelasert. Diese Erkennung wäre ebenso mittels Kamera auslesbar, ohne dass das Produkt einen Datenträger, wie einen RFID Chip, beinhaltet.

Gemäß einer bevorzugten Weiterbildung der unmittelbar vorstehenden beiden Ausführungsformen ist in dem dritten Modul und in dem vierten Modul eine künstliche Intelligenz vorgesehen. Mittels der künstlichen Intelligenz ist es vorgesehen, den Abgleich mit einem Sollzustand eines medizinischen Produkts durchzuführen, auszuwerten und auf diese Weise etwaige Defekte/ Deformationen zu erkennen. Die Verbindung mit der künstlichen Intelligenz bietet die vorliegende Offenbarung ein Tool/ Werkzeug, um auf die Fragestellung der Lebensdauer eine direkte Antwort geben zu können.

In anderen Worten wird bevorzugt eine künstliche Intelligenz beschrieben, um Produkte anhand derer Geometrie zu erkennen und eine Produktzuordnung zu ermöglichen mit gegebenenfalls gleichzeitiger Beurteilung der Verschleißgrenzen von medizinischen Produkten und der daraus resultierenden Entsorgung, Erneuerung, Überarbeitung, Wartung und/ oder Neubeschaffung. Somit ist hiermit insbesondere die Lebensdauer von medizinischen Produkten beurteilbar.

Gemäß einer weiteren bevorzugten Ausführungsform ist ein fünftes Modul, vorzugsweise ein Quantumdot-Sensor, dazu vorgesehen und ausgebildet, mittels Farbunterschieden Daten eines medizinischen Produkts zu sichern. In anderen Worten kann mittels Quantumdot-Sensorik ähnlich wie bei einem Data Matrix Code anhand von Farbunterschieden Daten gesichert und somit Identifikationen durchgeführt werden.

Ferner betrifft die Offenbarung ein System mit einem Smart Port, einem Siebkorb und darin einbringbaren Instrumentenhaltern, welcher die medizinischen Produkte halten, wobei der Smart Port dazu vorgesehen und ausgebildet ist, Daten und Informationen des zumindest einen medizinischen Produkts in dem Siebkorb und/ oder den Instrumentenhaltern auszulesen und an ein Instrumentendatenmanagementsystem des Kunden, eine Cloud und/ oder an ein Instrumentendatenmanagementsystem des Herstellers zur Weiterverarbeitung zu übermitteln.

Darüber hinaus betrifft die vorliegende Offenbarung einen Roboterarm mit einem daran angebrachten oder darin integrierten Smart Port gemäß einem der vorstehenden Aspekte. In anderen Worten kann der Smart Port ausgeführt als ein auf einen Roboterarm aufgesetztes oder integral gestaltetes Modul ausgebildet sein. Ein solcher Roboter wird in der CSSD genutzt, um automatisch medizinische Produkte zu erkennen und Daten aus medizinischen Produkten auszulesen. Diese werden zur Verfügung gestellt und an eine weitere Datenverarbeitungseinheit weitergegeben.

Ein Vorteil ist, dass der Weg hin zu einer vollautomatisierten CSSD geebnet wird und somit Kosten eingespart werden können. Vorgesehen sind hierbei ganze Aufbereitungsstraßen, welche die medizinischen Produkte durchgehend - den ganzen Tag, das ganze Jahr (24/7/365) - aufbereiten.

Darüber hinaus ist eine weitere bevorzugte Nutzung des Smart Ports als Data Hub vorgesehen. In anderen Worten, wird in Mesh Netz unter der intelligenten Steuerungseinheit angestrebt, so dass diese vollständig untereinander kommunizieren und Daten austauschbar sind. Um eine Lösung zu etablieren, die einem Smart Tray funktioniert, wird der Smart Port dahingehend ausgebaut, dass dieser als zentrales Data Hub ausgestaltet wird, das heißt, dieser sammelt die Daten der intelligenten Steuerungseinheit mittels BLE (Bluetooth) oder anderer Funktechnologie auf und speichert diese in einem hauseigenen IMS und Datenbanken, oder in einer serverbasierten oder cloudbasierten oder blockchainbasierten Datenaufbewahrungslösung ab.

Dies alles geschieht, sobald eine intelligente Steuerungseinheit in die Reichweite des Smart Port gelangt. Sprich in jedem Aufbereitungszyklus mindestens einmal. Daten des Aufbereitungszyklus sind Zählstände von Zyklen, Artikelnummer, Seriennummer, die Zyklen selbst, LifeCycleCounter uvw. mehr. Diese Funktionalität soll ebenso bidirektional funktionieren. Das heißt, alle intelligenten Steuerungseinheit erhalten denselben Datenstand und werden immer wieder beim Ankommen an dem Smart Port aktualisiert.

Eine weitere bevorzugte Nutzung der vorliegenden Offenbarung ist wie folgt vorgesehen: Stand heute werden Verbrauchsartikel steril verpackt angeliefert, welche dann erfasst werden müssen. Ebenso gibt es in Kliniken einzeln verpackte Instrumente, welche gesondert verpackt und sterilisiert werden. Der Smart Port ist bevorzugt dazu vorgesehen und ausgebildet, die medizinischen Produkte durch die Sterilverpackung zu scannen, ohne die Sterilbarriere zu öffnen.

Zusammenfassend bedient die vorliegende Offenbarung folgende Schlüsselfunktionen:
- Sterilisation: In einem Sterilgutkreislauf ist ein zentraler Readingpoint/ Erfassungsort, vorzugsweise an einem zentralen Punkt, insbesondere die CSSD, im Klinikum etabliert, welcher alle medizinischen Produkte aufgrund der Verwendung von allen etablierten Technologien erkennt;
- Robotik: Weg zur halbautomatisierten Erkennung von medizinischen Produkten in der AEMP;
- Künstliche Intelligenz: Erkennung von medizinischen Produkten mittels Bilderkennung und Lernen des Systems, insbesondere Kleinstprodukte wie Diamantfräser mit einem Durchmesser von 0,5 mm;

Ferner ist es in der vorliegenden Offenbarung vorzugsweise vorgesehen, den Smart Port mit einer Anzahl von zwei, drei, vier, fünf oder mehr verschiedenen Modulen beliebig kombinierbar auszustatten.

Ferner ist es in der vorliegenden Offenbarung vorgesehen, dass der Smart-Port gemäß einem der vorstehenden Aspekte zur Verwendung als Erfassungs- und Auslesevorrichtung von medizinischen Produkten ausgebildet/ vorgesehenak ist.

### Kurzbeschreibung der Figuren

Fig. 1 ist eine Darstellung zur Veranschaulichung eines Auslesesystems für medizinische Produkte gemäß dem Stand der Technik;
Fig. 2 ist eine Darstellung eines Smart Ports;
Fig. 3 ist eine Darstellung zur Positionierung des Smart Ports;
Fig. 4 ist eine Darstellung zur Veranschaulichung eines Auslesesystems für medizinische Produkte mit dem Smart Port;
Fig. 5 ist eine Darstellung eines Data Matrix Codes;
Fig. 6 ist eine Darstellung eines Barcodes;
Fig. 7 ist eine Darstellung des zweiten Moduls mit einem NFC-Tag und einem RFID-Tag;
Fig. 8 ist eine Darstellung einer durchgeführten Oberflächenabtastung;
Fig. 9 zeigt die Baugröße und -form eines LIDAR-Sensors des dritten Moduls;
Fig. 10 ist die Darstellung zweier erfasster Bilder des vierten Moduls;
Fig. 11 ist die Darstellung des QUANTUMDOT-Sensors des fünften Moduls;
Fig. 12 ist eine Darstellung eines Roboterarms mit einem daran angebrachten bzw. darin integrierten Smart Ports;
Fig. 13 ist eine Darstellung des Roboterarms im Betrieb;
Fig. 14 ist eine Darstellung der Kommunikationsverbindungen des Smart Ports im Betrieb;
Fig. 15 ist eine Darstellung des Systems mit einem Smart Port, einem Siebkorb, und einem Instrumentendatenmanagementsystem des Kunden, einer Cloud und einem Instrumentendatenmanagementsystem des Herstellers;
Fig. 16 ist die Darstellung eines medizinischen Produkts in einer Sterilverpackung.

### Beschreibung der Ausführungsbeispiele

Nachstehend werden bevorzugte Ausführungsbeispiele der vorliegenden Offenbarung auf der Basis der zugehörigen Figuren beschrieben.

Fig. 1 wurde bereits vorstehend in der Beschreibungseinleitung beschrieben.

Fig. 2 ist eine Darstellung eines Smart Ports 1. In Fig. 2 wird im Speziellen der Smart Port 1/ das multifunktionale Lese- und Schreibegerät und dessen Technologien beschrieben. Der Smart Port 1 ist nur schematisch dargestellt und kann im modularen Aufbau auch anders ausgestaltet sein. Demnach wird in Fig. 2 eine schematische Darstellung des Smart Ports 1 gezeigt, in welchem alle Funktionen/ Erfassungstechnologien vereint sind. Die Lager der einzelnen Module kann auch auf eine andere Weise platziert werden, sofern die ergonomischen Anforderungen dies erfordern.

Der Smart Port 1 weist ein erstes Modul 6 auf, welches als ein Scanner ausgebildet ist, und dazu vorgesehen ist, einen Barcode und/ oder einen Data Matrix Code oder ein anderen 2D Code zu scannen bzw. zu lesen. Ferner weist der Smart Port 1 ein zweites Modul 9 auf, welches als NFC- und/ oder RFID-Modul ausgebildet ist, und dazu vorgesehen ist, zumindest einen NFC-Tag 10 und/ oder zumindest einen RFID-Tag 11 auszulesen und ggf. wieder zu beschreiben. Der Smart Port 1 weist ein drittes Modul 12 auf, welches mittels LIDAR Oberflächenabtastung 13 durchführt, und dazu vorgesehen ist, zumindest ein medizinisches Produkt 2 anhand dessen Geometrie zu erkennen und zu beurteilen. Darüber hinaus weist der Smart Port 1 ein viertes Modul 14 auf, welches als ein Kamerasystem ausgebildet ist, und dazu vorgesehen ist, zumindest ein Bild 15 eines medizinischen Produkts 2 zu erfassen und mittels KI (künstliche Intelligenz) auszuwerten. Der Smart Port 1 verfügt zudem über ein fünftes Modul 16, welches als ein Quantumdot-Sensor 27 ausgebildet ist, und dazu vorgesehen ist, mit Farbunterschieden Daten eines medizinischen Produkts 2 zu erfassen.

Die Form des Smart Ports 1 kann unterschiedlich ausgestaltet sein. In Fig. 2 ist der Smart Port 1 mit einem rechteckigen Gehäuse 23 ausgebildet. Das erste Modul 6 ist in dem Gehäuse 23 zurückversetzt, das zweite Modul 9 befindet sich auf der ebenen Oberfläche des Gehäuses 23 und das dritte, vierte und fünfte Modul 12, 16 und 14 sind in einem nach oben an geschrägten Aufbau 24 integriert.

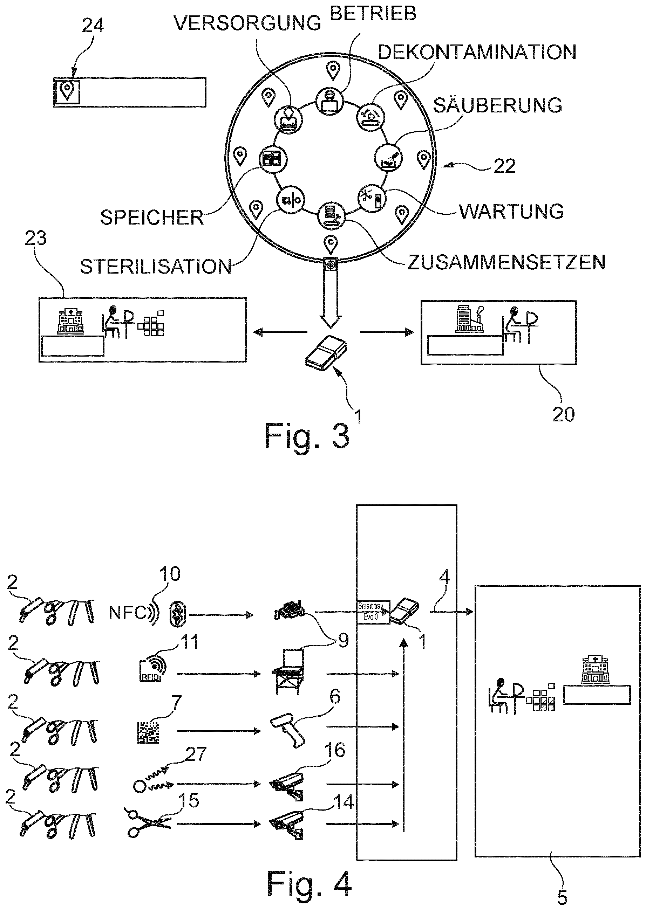

Fig. 3 ist eine Darstellung zur Positionierung des Smart Ports 1. Der Smart Port 1 steht an einem Packplatz, an welchem jedwedes genutzte medizinische Produkt 2 ohnehin in einem Zyklus 22 prozessiert wird. Bei einem solchen Zyklus 22 kann es sich um folgende Abteilungen handeln: ZSVA, AEMP, CSSD usw. An dieser Stelle werden die medizinischen Produkte 2 gesichtet, gepflegt und einem Siebkorb 18 zugeordnet. Der Smart Port 1 ist hierbei dazu ausgebildet, sich mit dem Instrumentendatenmanagementsystem des Herstellers 20 als auch mit dem Instrumentendatenmanagementsystem des Kunden 5 auszutauschen. Hierbei ist der Standort des Smart Ports 1 als Tracking Point 24 verfolgbar.

Fig. 4 ist eine Darstellung zur Veranschaulichung eines Auslesesystems für medizinische Produkte 2 mit dem Smart Port 1. Hierbei ist der Smart Port 1 in der Lage alle Erfassungstechnologien abzudecken. Mittels den im Smart Port 1 integrierbaren Modulen 6, 9, 12, 14 und 16 ist es möglich jedes medizinische Produkt 2 unabhängig von der Art der Produktmarkierung mit genau dem einen Smart Port 1 zu erfassen und die Daten an ein Instrumentendatenmanagementsystem des Kunden 5 weiterzugeben.

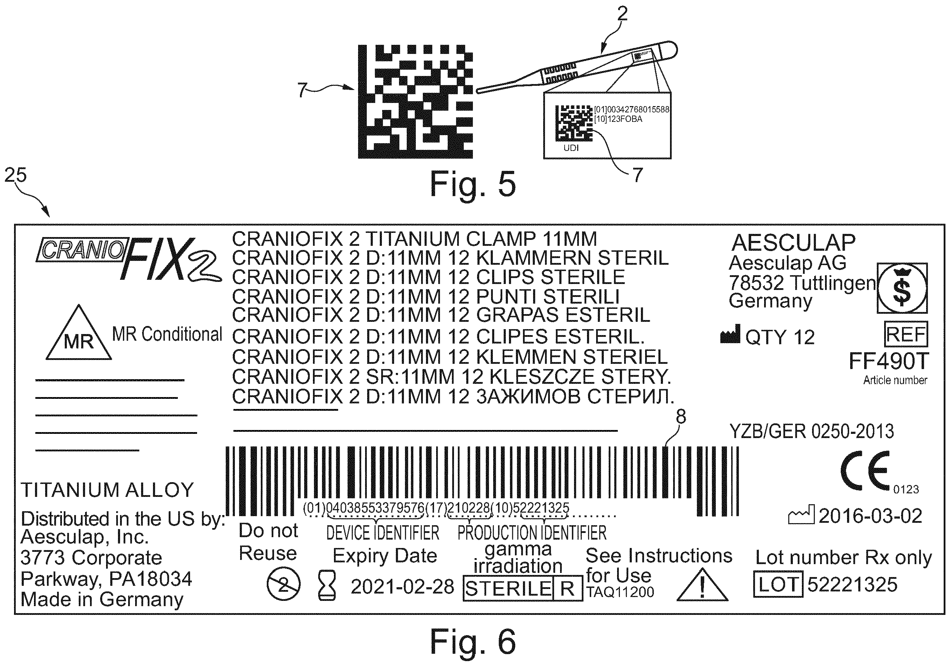

Fig. 5 ist eine Darstellung eines Data Matrix Codes 7. Der Data Matrix Code 7 ist in der rechten Hälfte von Fig. 5 auf einem medizinischen Produkt 2 angebracht und kann mittels dem ersten Modul 6 gemäß Fig. 2 ausgelesen werden.

Fig. 6 ist eine Darstellung eines Barcodes 8. Der Barcode 8 ist dazu vorgesehen auf ein Label 25 gedruckt zu werden und das Label 25 ist derart ausgebildet, um auf einem medizinischen Produkt 2 aufgebracht zu werden.

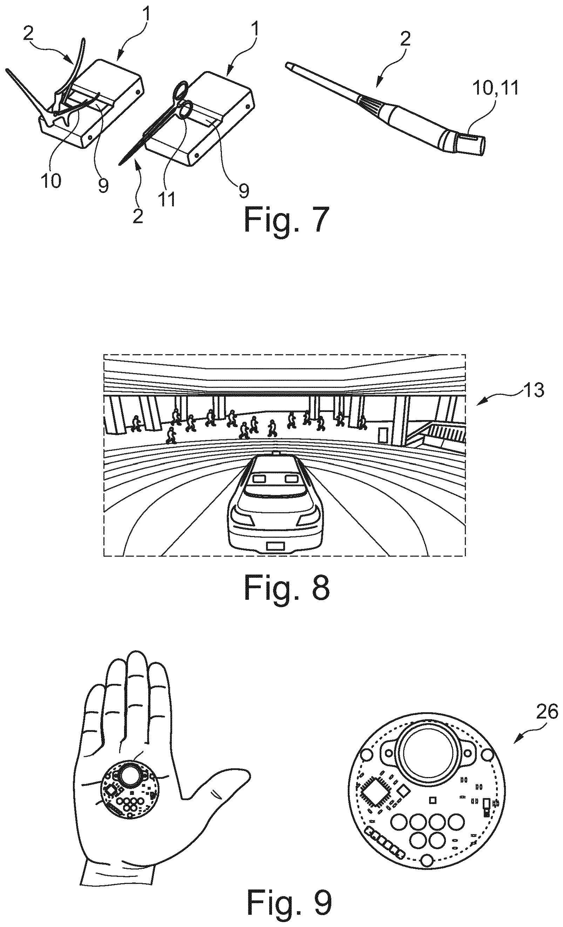

Fig. 7 ist eine Darstellung des zweiten Moduls 9 mit dem NFC-Tag 10 und dem RFID-Tag 11. In der linken Hälfte von Fig. 7 sind zwei Smart Ports 1 abgebildet, welche einmal beispielhaft zur Erkennung des NFC-Tags 10 und einmal zur Erkennung des RFID-Tags 11 an einem dargestellten medizinischen Produkt 2 gezeigt sind. In der rechten Hälfte von Fig. 7 ist ein beispielhaftes medizinisches Produkt 2 mit dem NFC-Tag 10 bzw. dem RFID-Tag 11 beispielshaft dargestellt.

Fig. 8 ist eine Darstellung einer durchgeführten Oberflächenabtastung 13 mit dem dritten Modul 12 gemäß Fig. 2.

Fig. 9 zeigt die Baugröße und -form eines LIDAR-Sensors 26 des dritten Moduls 12 des Smart Ports 1. Hierbei ist die Baugröße eines beispielhaften LIDAR-Sensors 26 etwa ein Fünftel einer Handinnenfläche.

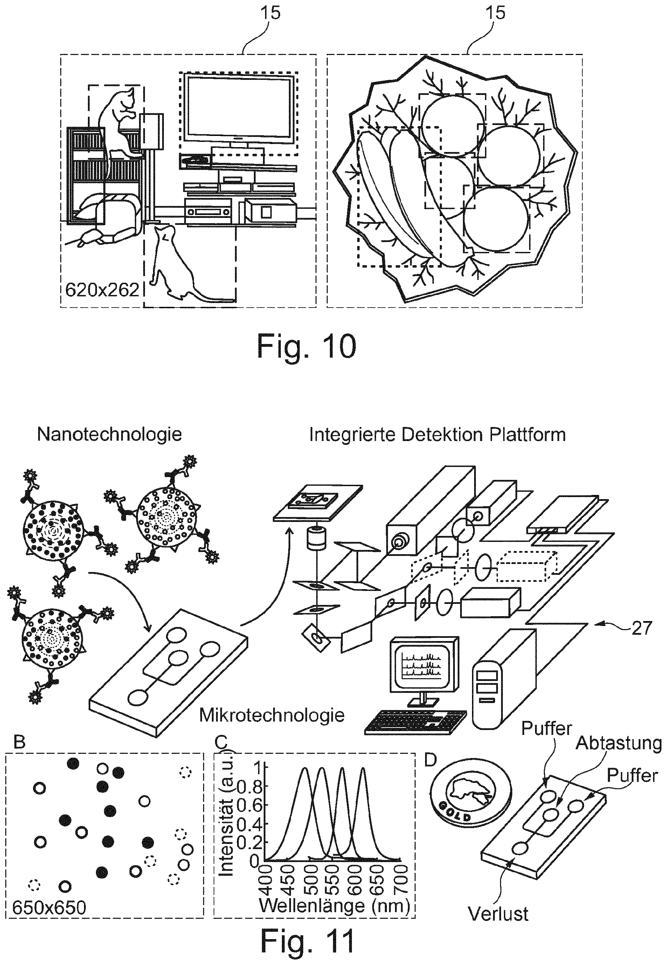

Fig. 10 ist die Darstellung zweier erfasster Bilder 15 des vierten Moduls 14 mit unterschiedlichen Gegenständen, deren Erkennung durch Umrandung kenntlich gemacht ist.

Fig. 11 ist die Darstellung des QUANTUMDOT-Sensors 27 des fünften Moduls 16.

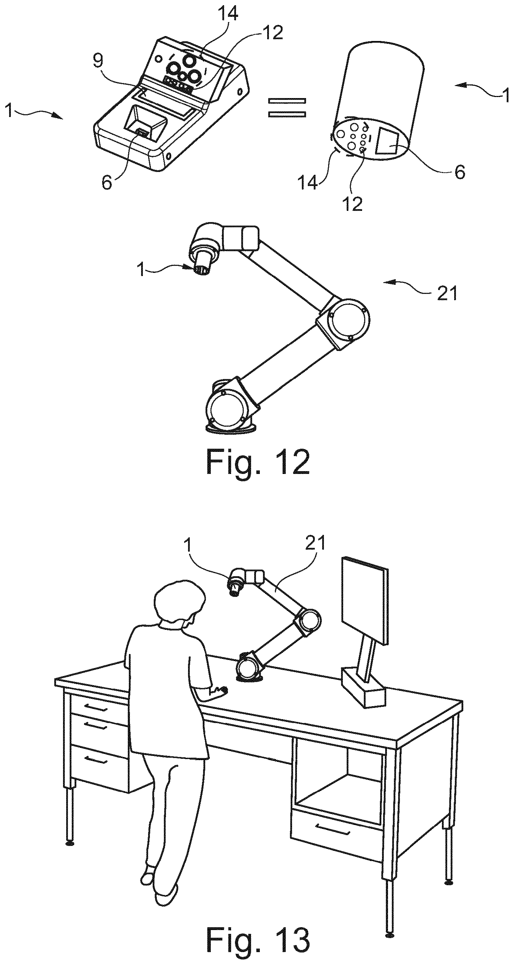

Fig. 12 ist eine Darstellung eines Roboterarms 21 mit einem daran angebrachten bzw. darin integrierten Smart Ports 1. In Fig. 12 ist gezeigt, dass der Smart Port 1 auch eine zylindrische Form aufweisen kann, wobei hierbei die Module 6, 9, 12, 14 und 16 in einer Stirnseite integriert sind. Der zylindrische Smart Port 1 ist einem Ende des Roboterarms 21 befestigt/ angebracht und dazu vorgesehen medizinische Produkte 2 zu erkennen/ erfassen.

Fig. 13 ist eine Darstellung des Roboterarms 21 im Betrieb.an

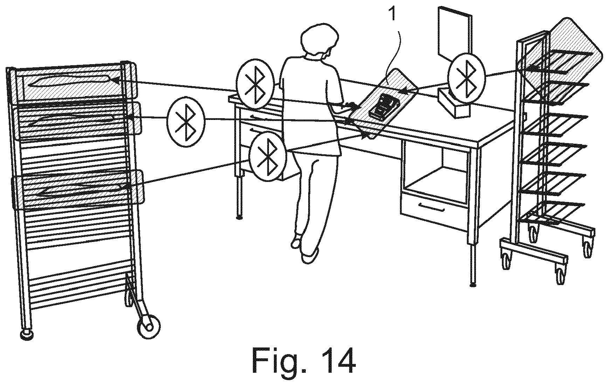

Fig. 14 ist eine Darstellung der Kommunikationsverbindungen 28 des Smart Ports 1 im Betrieb.

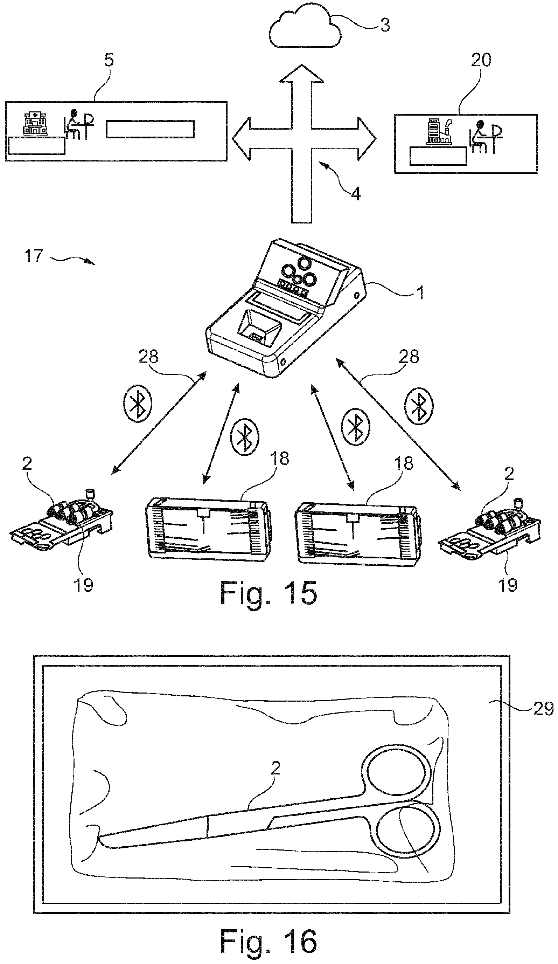

Fig. 15 ist eine Darstellung des Systems mit einem Smart Port 1, einem Siebkorb 18 und darin einbringbaren Instrumentenhaltern 19, welcher die medizinischen Produkte 2 halten, wobei der Smart Port 1 dazu vorgesehen und ausgebildet ist, Daten und Informationen des zumindest einen medizinischen Produkts 2 in dem Siebkorb 18 und/ oder den Instrumentenhaltern 19 auszulesen und an ein Instrumentendatenmanagementsystem des Kunden 5, eine Cloud 3 und/ oder an ein Instrumentendatenmanagementsystem des Herstellers 20 zur Weiterverarbeitung zu übermitteln. Ferner zeigt Fig. 15 die Kommunikationsverbindungen 28 per Bluetooth zwischen den medizinischen Produkten 2 bzw. den Siebkörben 18.

Fig. 16 ist die Darstellung eines medizinischen Produkts 2 in Form einer Schere in einer Sterilverpackung 29, welche gescannt werden kann, ohne die Sterilbarriere zu öffnen.

### Bezugszeichen

- 1: Smart Port
- 2: Medizinisches Produkt
- 3: Cloud
- 4: Schnittstelle
- 5: Instrumentendatenmanagementsystem des Kunden
- 6: Erstes Modul
- 7: Data Matrix Code
- 8: Barcode
- 9: Zweites Modul
- 10: NFC-Tag
- 11: RFID-Tag
- 12: Drittes Modul
- 13: Oberflächenabtastung
- 14: Viertes Modul
- 15: Bild
- 16: Fünftes Modul
- 17: System
- 18: Siebkorb
- 19: Instrumentenhalter
- 20: Instrumentendatenmanagementsystem des Herstellers
- 21: Roboterarm
- 22: Zyklus
- 23: Gehäuse
- 24: Aufbau
- 25: Label
- 26: LIDAR-Sensor
- 27: QUANTUMDOT-Sensors
- 28: Kommunikationsverbindungen
- 29: Sterilverpackung

## Patentansprüche

1. System (17) mit einem Smart Port (1), wobei der Smart Port (1) dazu vorgesehen und ausgebildet ist, Daten und Informationen von zumindest einen medizinischen Produkts (2) auszulesen und an ein Kunden-Instrumentendatenmanagementsystem (5), eine Cloud (3) und/ oder an ein Instrumentendatenmanagementsystem des System-Herstellers (20) zur Weiterverarbeitung zu übermitteln, wobei der Smart Port (1) zum einzelnen Erfassen und Auslesen von dem zumindest einen medizinischen Produkt (2) eine Vielzahl an separaten aber beliebig miteinander kombinierbaren Modulen aufweist, welche jeweils mit zueinander unterschiedlichen Erfassungs-Technologien vorgesehen und ausgebildet sind, um die Daten und Informationen des zumindest einen medizinischen Produkt (2) zu erfassen und auszulesen, wobei die Verwendung von zumindest einer optischen Erfassungstechnologie und parallel dazu einer Funkerfassungstechnologie vorgesehen ist, **dadurch gekennzeichnet, dass** der Smart Port (1), insbesondere eine Recheneinheit des Smart Ports (1) dazu vorgesehen und ausgebildet ist,
um innerhalb eines vorgegebenen und/oder vordefinierten Zeitfensters, das zumindest eine medizinische Produkt (2) zu erfassen und ein Signal zu versenden, wobei das Signal dazu vorgesehen ist, mit der Erfassung des medizinischen Produkts (2) eine richtige oder falsche Zuordnung zu protokollieren, und
innerhalb des Zeitfensters die optische Erfassungstechnologie und die Funkerfassungstechnologie gegeneinander abzugleichen, um die Richtigkeit der Zuordnung zu gewährleisten.

2. System (17) nach Anspruch 1, **dadurch gekennzeichnet, dass** der Smart-Port (1) einen um den Smart Port (1) liegenden Raum definiert, in welchem die medizinischen Produkte (2) auslesbar sind.

3. System (17) nach einem der Ansprüche 1 oder 2, **dadurch gekennzeichnet, dass** das Zeitfenster innerhalb von 2 bis 3 Sekunden beträgt.

4. System (17) nach einem der Ansprüche 1 bis 3, **dadurch gekennzeichnet, dass** der Smart Port (1) dazu vorgesehen und ausgebildet ist, um zumindest zwei unterschiedliche Feedback-Signale, insbesondere optisch und/oder akustisch, auszugeben, wodurch die richtige oder falsche Zuordnung erkennbar ist.

5. System (17) nach einem der Ansprüche 1 bis 4, **dadurch gekennzeichnet, dass** dieser dazu vorgesehen und ausgebildet ist, erfasste und ausgelesene Daten in eine Cloud (3) basierte Infrastruktur zu laden sowie mittels einer Schnittstelle (4) mit einem Instrumentendatenmanagementsystem des Kunden (5) und/ oder des Herstellers (20) zu kommunizieren.

6. System (17) nach Anspruch 5, **dadurch gekennzeichnet, dass** der Smart Port (1) als Datencollector fungiert und dazu vorgesehen und ausgebildet ist, die Daten bis zur Abrufung auf einem nicht flüchtigen Speicher, insbesondere auf einer SD-Karte, Festplatte und/oder USB-Stick zu sichern.

7. System (17) nach einem der Ansprüche 1 bis 6, **dadurch gekennzeichnet, dass** die Module ein erstes Modul (6) aufweisen, vorzugsweise ein Scanner, welches dazu vorgesehen und ausgebildet ist, einen Data Matrix Code (7) und/ oder einen Barcode (8) auszulesen, welcher dazu vorgesehen und ausgebildet ist, auf einem medizinischen Produkt (2) aufgebracht und auslesbar zu sein.

8. System (17) nach einem der Ansprüche 1 bis 6, **dadurch gekennzeichnet, dass** die Module ein zweites Modul (9) aufweisen, vorzugsweise ein RFID-Modul, insbesondere ein NFC-Modul, welches dazu vorgesehen und ausgebildet ist, RFID-Tag (11), insbesondere einen NFC-Tag (10) auszulesen, welcher dazu vorgesehen und ausgebildet ist, in einem medizinischen Produkt (2) untergebracht und auslesbar und/ oder beschreibbar zu sein.

9. System (17) nach Anspruch 8, **dadurch gekennzeichnet, dass** das zweite Modul (9) in einem Frequenzbereich von HF, UHF und/ oder SHF arbeitet.

10. System (17) nach einem der Ansprüche 1 bis 6, **dadurch gekennzeichnet, dass** die Module ein drittes Modul (12) aufweisen, vorzugsweise ein LIDAR-Sensor (26), welches dazu vorgesehen und ausgebildet ist, eine Oberflächenabtastung (13) von zumindest einem medizinischen Produkt (2) durchzuführen.

11. System (17) nach einem der Ansprüche 1 bis 6, **dadurch gekennzeichnet, dass** die Module ein viertes Modul (14) aufweisen, vorzugsweise ein BilderfassungsModul, welches dazu vorgesehen und ausgebildet ist, zumindest ein Bild (15) eines medizinischen Produkts (2) zu sichern.

12. System (17) nach einem der Ansprüche 1 bis 6, **dadurch gekennzeichnet, dass** ein Modul der Modulen dazu vorgesehen und ausgebildet ist, zumindest ein medizinisches Produkt (2) anhand dessen Geometrie zu erkennen und gegenüber einem Sollzustand des medizinischen Produkts (2) abzugleichen.

13. System (17) nach einem der Ansprüche 1 bis 6, **dadurch gekennzeichnet, dass** die Module ein fünftes Modul (16) aufweisen, vorzugsweise ein Quantumdot-Sensor (27), welches dazu vorgesehen und ausgebildet ist, mittels Farbunterschieden Daten eines medizinischen Produkts (2) zu sichern.

14. System (17) gemäß einem der Ansprüche 1 bis 13, **dadurch gekennzeichnet, dass** der Smart-Port (1) versehen ist mit einer Vorrichtung mit einer künstlichen Intelligenz zum Erkennen und Beurteilen von medizinischen Produkten (2) anhand der Geometrie der einzelnen medizinischen Produkte (2) sowie vorzugsweise das Bestimmen derer Lebensdauer.

15. System (17) gemäß einem der Ansprüche 1 bis 14 zur Verwendung als Erfassungs- und Auslesevorrichtung von medizinischen Produkten (2), vorzugsweise in einem Sterilgutkreislauf.

## Claims

1. A system (17) with a smart port (1), wherein the smart port (1) is provided and configured to read out data and information from at least one medical product (2) and to transmit it to a instrument data management system of a customer (5), a cloud (3) and/or to an instrument data management system of the system manufacturer (20) for further processing, wherein the smart port (1) has a plurality of separate but arbitrarily combinable modules for the individual acquisition and reading of the at least one medical product (2), wherein the modules are each provided and configured with respectively different detection technologies in order to detect and read out the data and information of the at least one medical product (2), wherein the use of at least one optical detection technology and in parallel thereto a radio detection technology is provided,
**characterized in that**
the smart port (1), in particular a computing unit of the smart port (1), is provided and configured,
to detect the at least one medical product (2) within a predetermined and/or predefined time window and to send a signal, wherein the signal is provided to log a correct or incorrect assignment with the detection of the medical product (2), and
to compare the optical detection technology and the radio detection technology within the time window in order to ensure the correctness of the assignment.

2. The system (17) according to claim 1, **characterized in that** the smart port (1) defines a space around the smart port (1) in which the medical products (2) can be read.

3. The system (17) according to one of claims 1 or 2, **characterized in that** the time window is within 2 to 3 seconds.

4. The system (17) according to one of claims 1 to 3, **characterized in that** the smart port (1) is provided and configured to output at least two different feedback signals, in particular optically and/or acoustically, whereby the correct or incorrect assignment can be recognized.

5. The system (17) according to one of claims 1 to 4, **characterized in that** it is provided and configured to load detected and read-out data into a cloud (3) based infrastructure and to communicate with an instrument data management system of a customer (5) and/or of a manufacturer (20) via an interface (4).

6. The system (17) according to claim 5, **characterized in that** the smart port (1) functions as a data collector and is provided and configured to save the data on a nonvolatile memory, in particular on an SD card, hard disk and/or USB stick, until it is retrieved.

7. The system (17) according to one of claims 1 to 6, **characterized in that** the modules have a first module (6), preferably a scanner, which is provided and configured to read a data matrix code (7) and/or a barcode (8), which is provided and configured to be applied to a medical product (2) and to be readable.

8. The system (17) according to one of claims 1 to 6, **characterized in that** the modules have a second module (9), preferably an RFID module, in particular an NFC module, which is provided and configured to read RFID tag (11), in particular an NFC tag (10), which is provided and configured to be accommodated in a medical product (2) and to be readable and/or writable.

9. The system (17) according to claim 8, **characterized in that** the second module (9) operates in a frequency range of HF, UHF and/or SHF.

10. The system (17) according to one of claims 1 to 6, **characterized in that** the modules have a third module (12), preferably a LIDAR sensor (26), which is provided and configured to perform surface scanning (13) of at least one medical product (2).

11. The system (17) according to one of claims 1 to 6, **characterized in that** the modules have a fourth module (14), preferably an image capture module, which is provided and configured to save at least one image (15) of a medical product (2).

12. The system (17) according to one of claims 1 to 6, **characterized in that** a module of the modules is provided and configured to detect at least one medical product (2) on the basis of its geometry and to compare it with a target state of the medical product (2).

13. The system (1) according to one of claims 1 to 6, **characterized in that** the modules have a fifth module (16), preferably a quantum dot sensor (27), which is provided and configured to save data of a medical product (2) via color differences.

14. The system (17) according to one of claims 1 to 13, **characterized in that** the smart port (13) is provided with an apparatus with artificial intelligence for recognizing and evaluating medical products (2) on the basis of the geometry of the individual medical products (2) and preferably for determining their service life.

15. The system (17) according to one of claims 1 to 14 for use as a detecting and reading apparatus of medical products (2), preferably in a sterile goods cycle.

## Revendications

1. Système (17) avec un port intelligent (1), dans lequel le port intelligent (1) est prévu et conçu pour lire des données et des informations d'au moins un produit médical (2) et les transmettre à un système de gestion de données d'instruments de client (5), à un cloud (3) et/ou à un système de gestion de données d'instruments du fabricant de système (20) pour un traitement ultérieur, dans lequel le port intelligent (1) présente, pour la saisie et la lecture individuelles du au moins un produit médical (2), une pluralité de modules séparés mais pouvant être combinés entre eux au choix qui sont respectivement prévus et conçus avec des technologies de détection différentes les unes des autres, pour détecter et lire les données et informations du au moins un produit médical (2), dans lequel l'utilisation d'au moins une technologie de détection optique et, en parallèle, d'une technologie de détection radio est prévue, **caractérisé en ce que** le port intelligent (1), en particulier une unité de calcul du port intelligent (1), est prévu et conçu,
pour détecter, à l'intérieur d'une fenêtre temporelle prédéterminée et/ou prédéfinie, le au moins un produit médical (2) et pour envoyer un signal, dans lequel le signal est prévu pour consigner une attribution correcte ou incorrecte à la détection du produit médical (2), et
à l'intérieur de la fenêtre temporelle, faire correspondre la technologie de détection optique et la technologie de détection radio entre elles afin de garantir l'exactitude de l'attribution.

2. Système (17) selon la revendication 1, **caractérisé en ce que** le port intelligent (1) définit un espace situé autour du port intelligent (1) dans lequel les produits médicaux (2) peuvent être lus.

3. Système (17) selon l'une quelconque des revendications 1 ou 2, **caractérisé en ce que** la fenêtre temporelle est comprise entre 2 et 3 secondes.

4. Système (17) selon l'une quelconque des revendications 1 à 3, **caractérisé en ce que** le port intelligent (1) est prévu et conçu pour émettre au moins deux signaux de rétroaction différents, en particulier optiques et/ou acoustiques, moyennant quoi l'attribution correcte ou incorrecte peut être détectée.

5. Système (17) selon l'une quelconque des revendications 1 à 4, **caractérisé en ce que** celui-ci est prévu et conçu pour charger des données saisies et lues dans une infrastructure basée sur le cloud (3) et pour communiquer au moyen d'une interface (4) avec un système de gestion des données des instruments du client (5) et/ou du fabricant (20).

6. Système (17) selon la revendication 5, **caractérisé en ce que** le port intelligent (1) fait office de collecteur de données et est prévu et conçu pour sauvegarder les données sur une mémoire non volatile, en particulier sur une carte SD, un disque dur et/ou une clé USB, jusqu'à ce qu'elles soient consultées.

7. Système (17) selon l'une quelconque des revendications 1 à 6, **caractérisé en ce que** les modules présentent un premier module (6), de préférence un scanner, qui est prévu et conçu pour lire un code de matrice de données (7) et/ou un code à barres (8) qui est prévu et conçu pour être appliqué et lu sur un produit médical (2).

8. Système (17) selon l'une quelconque des revendications 1 à 6, **caractérisé en ce que** les modules présentent un deuxième module (9), de préférence un module RFID, en particulier un module NFC, qui est prévu et conçu pour lire une étiquette RFID (11), en particulier une étiquette NFC (10), qui est prévue et conçue pour être logée dans un produit médical (2) et pour être lue et/ou inscriptible.

9. Système (17) selon la revendication 8, **caractérisé en ce que** le deuxième module (9) fonctionne dans une gamme de fréquences HF, UHF et/ou SHF.

10. Système (17) selon l'une quelconque des revendications 1 à 6, **caractérisé en ce que** les modules présentent un troisième module (12), de préférence un capteur LIDAR (26), qui est prévu et conçu pour effectuer un balayage de surface (13) d'au moins un produit médical (2).

11. Système (17) selon l'une quelconque des revendications 1 à 6, **caractérisé en ce que** les modules présentent un quatrième module (14), de préférence un module d'acquisition d'images, qui est prévu et conçu pour sauvegarder au moins une image (15) d'un produit médical (2).

12. Système (17) selon l'une quelconque des revendications 1 à 6, **caractérisé en ce qu'**un module des modules est prévu et conçu pour reconnaître au moins un produit médical (2) à l'aide de sa géométrie et pour le comparer par rapport à un état de consigne du produit médical (2).

13. Système (17) selon l'une quelconque des revendications 1 à 6, **caractérisé en ce que** les modules présentent un cinquième module (16), de préférence un capteur à point quantique (27), qui est prévu et conçu pour sauvegarder des données d'un produit médical (2) au moyen de différences de couleur.

14. Système (17) selon l'une quelconque des revendications 1 à 13, **caractérisé en ce que** le port intelligent (1) est doté d'un dispositif avec une intelligence artificielle pour reconnaître et évaluer des produits médicaux (2) à l'aide de la géométrie des différents produits médicaux (2) ainsi que, de préférence, déterminer leur durée de vie.

15. Système (17) selon l'une quelconque des revendications 1 à 14, destiné à être utilisé en tant que dispositif de détection et de lecture de produits médicaux (2), de préférence dans un circuit de stérilisation.
